# FASCICULE DE BREVET EUROPEEN

(11) **EP 3 668 429 B1**
(45) Date de publication et mention de la délivrance du brevet: **16.06.2021**
(21) Numéro de dépôt: 18769756.0
(22) Date de dépôt: 16.08.2018
(51) Int. Cl.: A61B 17/88, A61B 17/70

(54) **ENSEMBLE D'ASSISTANCE AU POSITIONNEMENT D'UN IMPLANT INTERVERTÉBRAL ET KIT CHIRURGICAL L'INCORPORANT**
ANORDNUNG ZUR UNTERSTÜTZUNG DER POSITIONIERUNG EINES ZWISCHENWIRBELIMPLANTATS UND DIESEN ENTHALTENDES CHIRURGISCHES KIT
ASSEMBLY FOR ASSISTING WITH THE POSITIONING OF AN INTERVERTEBRAL IMPLANT AND SURGICAL KIT INCORPORATING THE SAME

(30) Priorité: 16.08.2017 FR 1757698
(43) Date de publication de la demande: 24.06.2020
(73) Titulaire: Backbone, 33110 Le Bouscat (FR)
(72) Inventeur: LUTZ, Christian, 24148 Kiel (DE)
(74) Mandataire: Plasseraud IP
(86) Numéro de dépôt international: PCT/FR2018/052068
(87) Numéro de publication internationale: WO 2019/034825

(56) Documents cités:
- EP-A1- 2 184 023
- EP-A1- 2 515 778

## Description

### DOMAINE TECHNIQUE

La présente invention se rapporte de manière générale à un ensemble d'assistance au positionnement d'un implant intervertébral ainsi qu'à un kit chirurgical comprenant un implant intervertébral ainsi que son ensemble d'assistance au positionnement.

### ETAT DE LA TECHNIQUE

Les opérations dans le domaine de la chirurgie du rachis peuvent concerner la région cervicale (nuque), la région dorsale ou, plus fréquemment, la région lombaire.

Lorsqu'il existe une instabilité, comme par exemple un glissement d'une vertèbre aux vertèbres adjacentes, une opération de stabilisation de la colonne vertébrale peut consister à implanter des implants intervertébraux.

Ces implants constituent un échafaudage qui joue un rôle de stabilisateur de la colonne vertébrale EP 2 515 778 décrit de tels implants.

La plupart des opérations lombaires sont effectuées en foyer ouvert par l'arrière (voie d'abord postérieure), en pratiquant une incision dans le dos du patient au niveau des vertèbres à stabiliser.

La conception des implants de l'art antérieur implique la réalisation de gestes par le chirurgien qui nécessitent de dégager une zone d'intervention assez large autour des vertèbres à stabiliser, notamment pour la mise en place du ou des liens souples dans la cale, et pour la mise en tension et le blocage de ce ou de ces liens.

Si des kits chirurgicaux comprenant un implant intervertébral et un porte-implant permettant la mise en place d'un implant suivant l'axe de la voie d'abord postérieure seulement ont été décrits dans l'état de la technique, la mise en place du ou des liens souples dans la cale ainsi que la mise en tension et le serrage sur le lien souple sont réalisés de manière latérale provoquant notamment un délabrement musculaire.

Les termes « postérieure » et « latérale » sont ceux du vocabulaire utilisé dans le domaine de la chirurgie du rachis.

Il importe donc de procurer des instruments chirurgicaux pour la mise en place d'implants intervertébraux ainsi que pour la mise en tension et le serrage de liens dans le contexte opératoire, qui permettent de réduire la taille de l'incision au strict minimum. En effet, il convient de protéger les tissus environnants (notamment les tissus musculaires qui participent à la stabilité de la colonne vertébrale) du stress lié à l'écartement de la plaie chirurgical qui peut entrainer des complications postopératoires jusqu'à des nécroses sévères.

Il existe ainsi un besoin de mettre à disposition un ensemble d'assistance au positionnement d'un implant intervertébral permettant de mettre en place et de serrer le lien souple dudit implant selon une voie d'abord postérieure.

### OBJET DE L'INVENTION

La présente invention concerne un ensemble d'assistance au positionnement d'un implant intervertébral,
ledit implant comprenant une cale de stabilisation adaptée pour stabiliser entre elles au moins deux vertèbres adjacentes par interposition entre des apophyses épineuses des vertèbres, et au moins une tresse souple pour la fixation de la cale de stabilisation aux apophyses épineuses des vertèbres à stabiliser, ladite tresse comprenant une extrémité libre,
ledit ensemble comprenant un porte-implant ayant un corps allongé s'étendant suivant un axe longitudinal entre des première et deuxième extrémités, la première extrémité étant adaptée pour être fixée à la cale de stabilisation,
caractérisé en ce que ladite première extrémité dudit porte-implant comporte un dispositif de renvoi comprenant une ouverture ayant un axe central sensiblement perpendiculaire à l'axe longitudinal du porte-implant mais non-concourant avec ledit axe longitudinal, ladite ouverture permettant ainsi le passage de ladite tresse de façon que, en utilisation, ladite tresse puisse présenter un première partie s'étendant sensiblement perpendiculairement à l'axe longitudinal depuis ledit implant jusqu'à ladite ouverture, et une deuxième partie s'étendant depuis ladite ouverture jusqu'à ladite extrémité libre dans un plan sensiblement parallèle à l'axe longitudinal du porte-implant pour permettre la mise en tension de ladite tresse.

Ces dispositions permettent de minimiser l'invasivité du mode opératoire, de diminuer la durée de l'opération chirurgicale, qui peut avantageusement être de type ambulatoire, de préserver les tissus et de réduire la zone d'intervention.

Dans des modes de réalisation particuliers de l'ensemble d'assistance, on peut avoir recours à l'une et / ou à l'autre des dispositions suivantes :
- le dispositif de renvoi comprend une paroi de guidage qui comporte ladite ouverture, ladite paroi de guidage s'étendant dans un plan moyen qui est sensiblement parallèle audit axe longitudinal mais qui ne contient pas ledit axe longitudinal.
- la paroi de guidage comporte une face avant orientée généralement à l'opposé du porte-implant, ladite face avant s'étendant latéralement entre un premier bord relativement éloigné du porte-implant et un deuxième bord plus proche du porte-implant, le deuxième bord de la face avant étant pourvu d'un renflement en saillie vers l'avant, qui permet de renvoyer ladite première partie de la tresse vers l'ouverture.
- le système de renvoi comprend en outre un support solidaire du porte-implant et portant la paroi de guidage en porte-à-faux par rapport audit porte-implant.
- le support comporte une première paroi solidaire du porte-implant et s'étendant sensiblement perpendiculairement à l'axe longitudinal du porte-implant, et une deuxième paroi reliant la première paroi à la paroi de guidage, ladite deuxième paroi étant sensiblement perpendiculaire à la première paroi et à la paroi de guidage, ledit renflement s'étendant sensiblement à l'opposé de la deuxième paroi.
- le corps allongé du porte-implant est de forme tubulaire avec un canal interne s'étendant entre les deux extrémités du porte-implant suivant l'axe longitudinal du corps allongé du porte-implant.
- la deuxième extrémité du porte-implant comprend un dispositif de mise en tension permettant de mettre en tension ladite tresse.
- le dispositif de mise en tension comprend un tambour configuré pour enrouler ladite tresse et une poignée permettant de faire tourner ledit tambour.
- la poignée est reliée au tambour par un moyen pour limiter le couple que la poignée peut transmettre au tambour.
- la deuxième extrémité du porte-implant comporte en outre un manche.

Par ailleurs, l'invention concerne également un kit chirurgical comprenant un implant intervertébral et un ensemble d'assistance au positionnement permettant une mise en position de l'implant dans le plan postérieur strict de l'implant et de la plaie chirurgicale, ledit implant comprenant
- une cale de stabilisation adaptée pour stabiliser entre elles au moins deux vertèbres adjacentes par interposition entre des apophyses épineuses des vertèbres, ayant un corps sensiblement parallélépipédique et comprenant une face supérieure et au moins une partie latérale présentant un axe principal déterminé, dans lequel est prévu un évidement présentant un axe longitudinal déterminé parallèle à l'axe principal du corps de la cale de stabilisation, une paroi interne s'étendant parallèlement à l'axe longitudinal de l'évidement, et une zone d'entrée taraudée ;
- au moins une tresse formant un lien souple pour la fixation de la cale de stabilisation aux apophyses épineuses des vertèbres à stabiliser, ladite au moins une tresse présentant une première extrémité fixée à ladite cale de stabilisation et une seconde extrémité libre ;

Dans des modes de réalisation particuliers du kit chirurgical, on peut avoir recours à l'une et / ou à l'autre des dispositions suivantes :
- le porte-implant présente à sa première extrémité un filet adapté pour coopérer avec le taraudage de la zone d'entrée de l'évidement pour la fixation par vissage du porte-implant à ladite cale de stabilisation de manière à ce que l'axe longitudinal du porte-implant coïncide avec l'axe longitudinal de l'évidement et que la paroi de guidage du dispositif de renvoi comportant ladite ouverture s'étende dans un plan moyen qui est sensiblement parallèle audit axe longitudinal mais qui ne contient pas ledit axe longitudinal, de façon à ce que la tresse lors de son passage dans l'ouverture puisse présenter une première partie s'étendant sensiblement perpendiculairement à l'axe longitudinal depuis ledit implant jusqu'à ladite ouverture, et une deuxième partie s'étendant depuis ladite ouverture jusqu'à ladite extrémité libre dans un plan sensiblement parallèle à l'axe longitudinal du porte-implant pour permettre la mise en tension de ladite tresse.
- le deuxième bord de la face arrière de la paroi de guidage est plaqué contre le bord rostral de la cale de stabilisation, de manière à ce que le renflement du deuxième bord de la face avant de la paroi de guidage soit en saillie vers l'avant, de façon à renvoyer ladite première partie de la tresse vers l'ouverture.
- le kit chirurgical comprend en outre une tige d'insertion d'un pion de blocage ayant une première extrémité et une seconde extrémité adaptée pour coulisser dans le canal interne du corps tubulaire du porte-implant pour l'insertion et le guidage d'un pion de blocage à travers le canal interne du porte-implant jusque dans l'évidement prévu dans le corps de la cale de stabilisation de manière à bloquer en mouvement par rapport à la cale de stabilisation par pincement ladite tresse entre le pion de blocage et les portions respectives de la paroi interne de l'évidement se faisant face l'une à l'autre, lorsque ladite tresse a été mise en tension via le dispositif de mise en tension de l'ensemble d'assistance au positionnement.
- le kit chirurgical comprend en outre un verrouilleur d'implant ayant un corps allongé s'étendant suivant un axe longitudinal entre des première et deuxième extrémités, ledit verrouilleur étant adapté pour coulisser dans le canal interne du corps tubulaire du porte implant, ladite première extrémité étant adaptée pour être fixée à la cale de stabilisation et ladite seconde extrémité étant adaptée pour prendre appui sur au moins une partie du porte-implant.

Préférentiellement, ledit verrouilleur d'implant présente à sa première extrémité un filet adapté pour coopérer avec le taraudage de la zone d'entrée de l'évidemment.

Préférentiellement, la seconde extrémité comprend un épaulement.

Avantageusement, le verrouilleur d'implant est vissé jusqu'en butée dans la cale et permet de sécuriser l'assemblage du porte-implant et de la cale de stabilisation de manière à ce que l'axe longitudinal du porte implant coïncide avec l'axe longitudinal de l'évidemment et ce durant toute la durée de l'opération.
- Le kit chirurgical comprend un pion de blocage ou verrou d'un seul tenant ayant une forme conique ou sensiblement conique.

Selon un mode de réalisation, l'une des extrémités du pion de blocage ou verrou est de forme conique ou sensiblement conique et permet de pincer la tresse entre le verrou et les portions respectives de la paroi interne de l'évidement se faisant face l'une à l'autre lorsque la tresse est mise en tension.

Avantageusement, le pincement de la tresse permet d'assurer son blocage une fois la tresse immobilisée en position avec la tension adéquate sans risque d'endommagement des fibres de la tresse.

Dans un mode de réalisation préféré, le pion de blocage comprend, à l'opposé de son extrémité conique, une extrémité présentant un filet adapté pour coopérer avec le taraudage de la zone d'entrée de l'évidement afin de visser ledit pion de blocage dans l'évidement de la cale de stabilisation jusqu'en butée.
- Le kit chirurgical comprend une seconde cale de stabilisation, ladite ou un desdites tresses ayant une dimension longitudinale permettant son insertion dans les deux cales de stabilisation lorsque chacune de ces cales est placée entre deux apophyses.

De cette façon, ledit kit chirurgical permet de stabiliser entre elles trois vertèbres consécutives par interposition de deux cales de stabilisation entre les apophyses épineuses desdites trois vertèbres adjacentes.

De manière préférée, les deux cales de stabilisation sont reliées par une même tresse.

La présente invention concerne également un implant vertébral selon la présente invention pour son utilisation dans le traitement des lésions dégénératives lombaires, préférentiellement des lésions dégénératives lombaires de grade II ou III ou IV selon la classification IRM de Pfirmann.

Préférentiellement encore, les lésions dégénératives lombaires sont situées de L1 à L5.

La présente invention concerne également une méthode de traitement de lésions dégénératives lombaires, préférentiellement des lésions dégénératives lombaires de grade II ou III ou IV selon la classification IRM de Pfirmann comprenant l'utilisation d'un kit chirurgical selon la présente invention.

Préférentiellement, les lésions dégénératives lombaires sont situées de L1 à L5.

Typiquement, les lésions dégénératives limbaires pourront être choisies parmi la hernie discale volumineuse, la récidive d'hernie discale ou hernie discale sur anomalie transitionnelle par sacralisation de L5 traitées par discectomie, la discopathie dégénérative sur un niveau adjacent à une fusion lombo-sacrée, la lésion dégénérative avec ou sans lésions de type Modic 1, le canal lombaire étroit traité par laminectomie.

Avantageusement, le kit chirurgical selon l'invention permet de minimiser l'invasivité du mode opératoire et permettre de mettre en place un implant intervertébral et mettre en tension le lien souple selon une voie d'abord postérieure afin de préserver les tissus et réduire la zone d'intervention.

### BREVE DESCRIPTION DES DESSINS

D'autres caractéristiques et avantages de l'invention apparaîtront encore à la lecture de la description qui va suivre. Celle-ci est purement illustrative et doit être lue au regard des dessins annexés sur lesquels :
- La Figure 1, est une vue en trois dimensions d'un ensemble d'assistance au positionnement selon des modes de réalisation ;
- La Figure 2, est une vue en trois dimensions d'un ensemble d'assistance au positionnement selon des modes de réalisation, montrant en outre la face avant de la paroi de guidage pourvue d'un renflement en saillie vers l'avant et un dispositif de mise en tension ;
- La Figure 3, est une vue éclatée en trois dimensions d'une partie de l'implant intervertébral selon des modes de réalisation ;
- La Figure 4, est une vue de face, suivant l'axe de la voie d'abord postérieur, de l'implant selon des modes de réalisation ;
- La Figure 5, est une vue en trois dimensions d'une partie de l'implant intervertébral et d'un ensemble d'assistance au positionnement associé, selon des modes de réalisation ;
- La Figure 6, est une vue en trois dimensions d'une partie de l'implant intervertébral et d'un ensemble d'assistance au positionnement associé, selon des modes de réalisation, montrant en outre la face avant de la paroi de guidage pourvue d'un renflement en saillie vers l'avant ;
- Les Figures 7 et 8, sont des vues en trois dimensions d'une partie de l'implant intervertébral et d'un ensemble d'assistance au positionnement associé, selon des modes de réalisation, montrant en outre une première partie de la tresse s'étendant sensiblement perpendiculairement à l'axe longitudinal du porte-implant depuis l'implant jusqu'à l'ouverture du dispositif de renvoi et une deuxième partie s'étendant depuis l'ouverture jusqu'à son extrémité libre dans un plan sensiblement parallèle à l'axe longitudinal du porte-implant ;
- La Figure 9, est une vue en trois dimensions d'une partie de l'implant intervertébral et d'un ensemble d'assistance au positionnement associé, selon des modes de réalisation, montrant en outre le dispositif de mise en tension.
- La Figure 10 est une vue en trois dimension d'une partie de l'implant intervertébral, d'un ensemble d'assistance au positionnement et du verrouilleur d'implant selon des modes de réalisation.

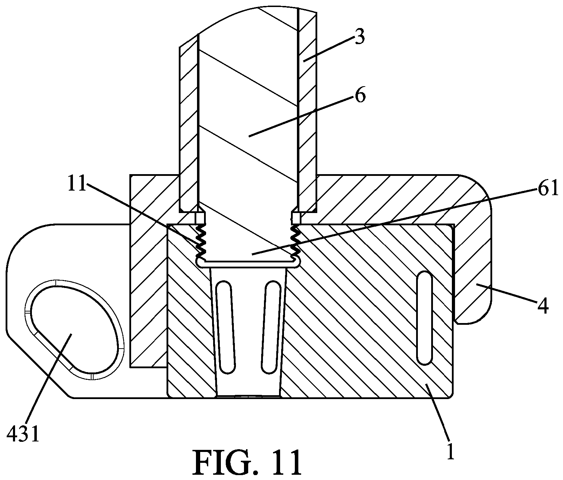

- La Figure 11 est une vue en coupe d'une partie de l'implant intervertébral, d'une partie d'un ensemble d'assistance au positionnement et d'une partie du verrouilleur d'implant selon des modes de réalisation.

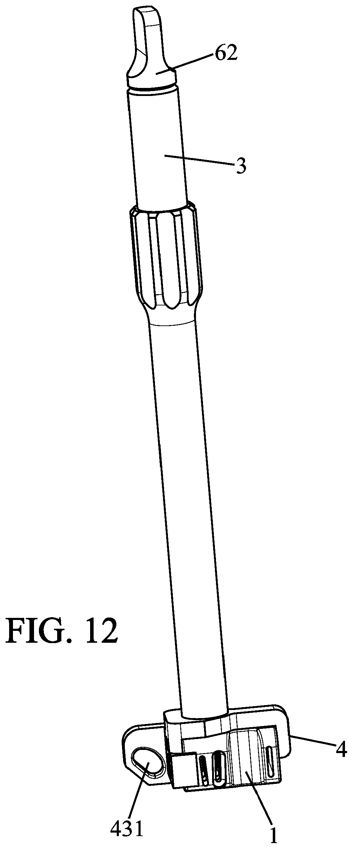

- La Figure 12 est une vue en trois dimension d'une partie de l'implant intervertébral, d'un ensemble d'assistance au positionnement et du verrouilleur d'implant selon des modes de réalisation.
- La Figure 13 est une vue en trois dimensions d'une partie de la cale de stabilisation, d'un pion de blocage ou verrou et de la tresse ou lien souple.
- La Figure 14 est une vue en trois dimensions d'une partie de la cale de stabilisation, d'un pion de blocage ou verrou et de la tresse ou lien souple, le pion de blocage étant en position verrouillée.
- La Figure 15 est une vue en coupe de la cale de stabilisation, d'un pion de blocage ou verrou et de la tresse ou lien souple, le pion de blocage étant en position verrouillée.
- La Figure 16 est une vue en coupe d'une partie d'un porte-implant et d'un verrouilleur d'implant.

### DESCRIPTION DETAILLEE DE MODES DE REALISATION

L'ensemble d'assistance selon la présente invention est destiné à positionner un implant vertébral entre les apophyses épineuses de deux vertèbres adjacentes, c'est-à-dire des vertèbres consécutives dans l'empilement des vertèbres lombaires, dorsales et cervicales.

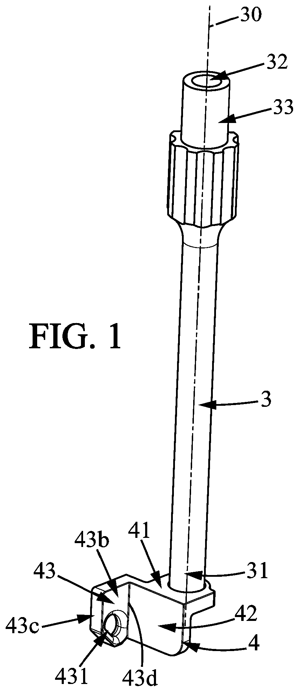

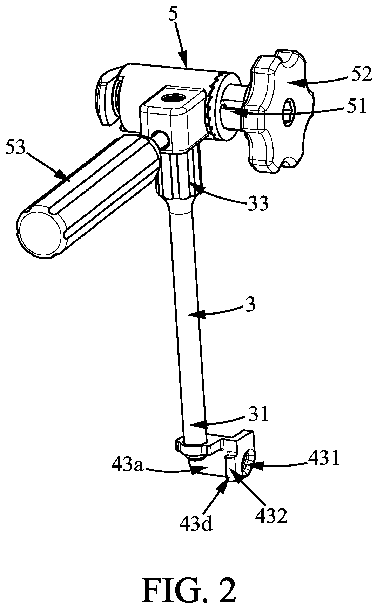

Les principaux éléments de l'ensemble d'assistance au positionnement de l'implant intervertébral selon des modes de réalisation de la présente invention vont être décrits, tout d'abord, en référence aux Figures 1 et 2. Ainsi qu'il est montré sur ces figures, l'ensemble d'assistance est composé d'un porte-implant 3, d'un dispositif de renvoi 4 et d'un dispositif de mise en tension 5.

Ainsi qu'il est visible sur la Figure 1, le porte-implant 3 a un corps allongé s'étendant suivant un axe longitudinal 30 entre des première et deuxième extrémités 31, 33.

Le corps allongé du porte-implant 3 est de forme tubulaire avec un canal interne 32 s'étendant entre les deux extrémités 31, 33 suivant l'axe longitudinal 30 du corps allongé du porte-implant.

La première extrémité 31 du porte-implant comporte un dispositif de renvoi 4 comprenant une première partie 41 solidaire du porte-implant 3 s'étendant sensiblement perpendiculairement à l'axe longitudinal 30 du porte implant, une deuxième paroi 42, et une paroi de guidage 43.

La première partie 41 et la deuxième partie 42 sont adaptées pour être plaquées sur le corps de la cale de stabilisation de l'implant et plus particulièrement pour maintenir en position le dispositif de renvoi 4 sur la cale de stabilisation de l'implant.

La deuxième paroi 42 relie la première paroi 41 à la paroi de guidage 43 et est sensiblement perpendiculaire à la première paroi 41 et à la paroi de guidage 43.

La paroi de guidage 43 s'étend dans un plan moyen qui est sensiblement parallèle à l'axe longitudinal 30 du porte-implant mais qui ne contient pas cet axe longitudinal 30.

La paroi de guidage 43 comporte une face avant 43a et une face arrière 43b, s'étendant entre un premier bord 43c et un second bord 43d. Le premier bord 43c est relativement éloigné du porte-implant 3 et le second bord 43d est plus proche du porte-implant 3.

La paroi de guidage 43 comprend une ouverture 431 ayant un axe central sensiblement perpendiculaire à l'axe longitudinal 30 du porte-implant 3 mais non-concourant avec ledit axe longitudinal 30.

L'ouverture 431 permet le passage d'une tresse 2 d'un implant de façon que, en utilisation, la tresse 2 puisse présenter un première partie s'étendant sensiblement perpendiculairement à l'axe longitudinal 30 depuis ledit implant jusqu'à ladite ouverture, et une deuxième partie s'étendant depuis ladite ouverture jusqu'à ladite extrémité libre 21 dans un plan sensiblement parallèle à l'axe longitudinal 30 du porte-implant 3 pour permettre la mise en tension de ladite tresse 2.

Tel qu'illustré notamment sur la Figure 2, le deuxième bord 43d de la face avant 43a de la paroi de guidage 43 est pourvu d'un renflement 432 en saillie vers l'avant, qui permet de renvoyer la tresse 2 de l'implant vers l'ouverture 431.

La deuxième extrémité 33 du porte-implant 3 comprend un dispositif de mise en tension 5 permettant de mettre en tension la tresse 2 d'un implant.

Ce dispositif de mise en tension 5 comprend un tambour 51, une poignée 52 et un manche 53 pour faciliter la manipulation du dispositif de mise en tension.

La fonction de la poignée 52 est de faire tourner le tambour de manière à pouvoir enrouler la tresse 2 reçue dans le tambour pour, une fois l'implant positionné entre les apophyses épineuses de deux vertèbres adjacentes, que la tresse soit mise en tension afin de stabiliser l'implant entre les apophyses épineuses de vertèbres.

Le dispositif de mise en tension 5 peut être relié à un moyen (non représenté) permettant de limiter le couple que la poignée peut transmettre au tambour. Typiquement, le moyen peut être une clé dynamométrique.

Typiquement, la tension maximale pouvant être appliquée aux apophyses épineuses de vertèbres est d'environ 400N.

L'ensemble d'assistance au positionnement peut être réalisé dans tout matériau à usage médical, par exemple l'inox.

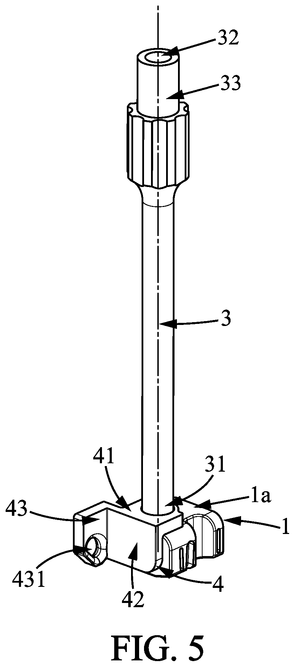

Les Figures 5 à 9 décrivent un kit chirurgical comprenant un ensemble d'assistance au positionnement tel qu'illustré à la Figure 1 ainsi qu'un implant intervertébral.

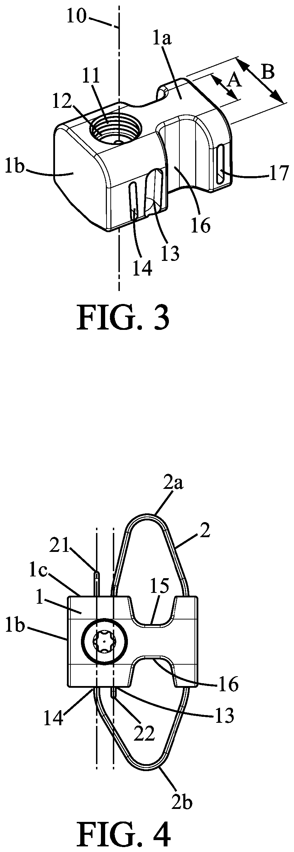

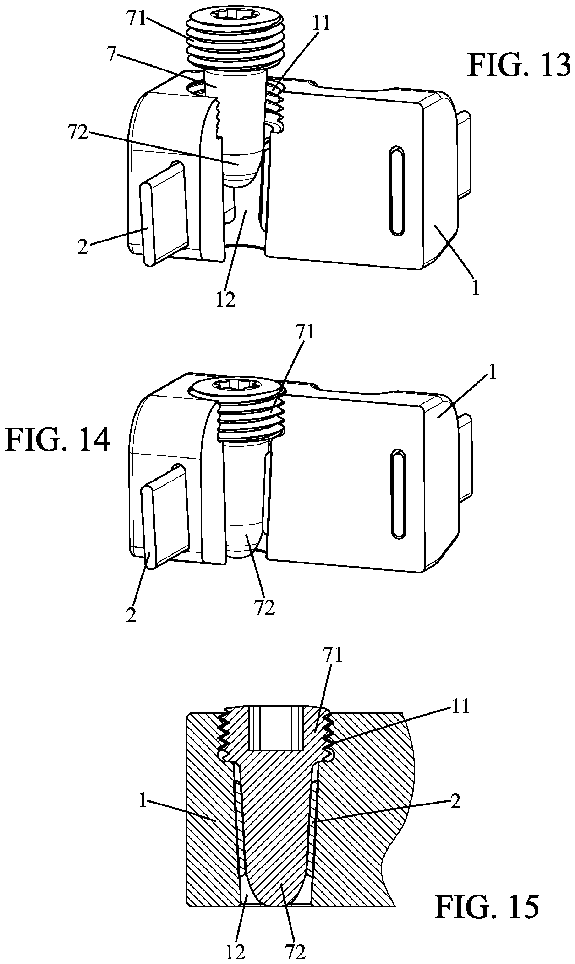

L'implant vertébral selon des modes de réalisation de la présente invention va être décrit, tout d'abord, en référence aux Figures 3 et 4. Ainsi qu'il est montré sur ces figures, l'implant est composé d'une cale de stabilisation 1 et d'une tresse 2. L'implant intervertébral comprend en outre un pion de blocage ou verrou 7, représenté aux Figures 13 à 15.

La cale de stabilisation 1 comprend un corps globalement parallélépipédique avec un axe principal qui, pour des raisons de clarté de la Figure 3, est confondu sur cette figure avec l'axe longitudinal 10 d'un évidement 12 prévu dans le corps. La Figure 4 est une vue de l'implant suivant l'axe longitudinal 10, lorsque l'implant est posé à plat contre les vertèbres du patient (lequel est allongé sur la table d'opération, sur le ventre ou décubitus ventral). L'axe principal 10 coïncide alors avec l'axe de la voie d'abord postérieure, c'est-à-dire qu'il est perpendiculaire au dos du patient et donc à l'axe de la colonne vertébrale correspondant à la direction d'empilement des vertèbres depuis les vertèbres lombaires jusqu'aux vertèbres cervicales.

Pour ce qui est du vocabulaire descriptif, on considère dans la suite la direction d'observation du site d'implantation par le chirurgien suivant l'axe de la voie d'abord postérieure, lors de l'opération d'implantation et alors que le patient est allongé sur le ventre contre la table d'opération. Ainsi, la Figure 4 représente une vue de face suivant cet axe, et une vue de dessus suivant cette direction. Les termes « antérieur » et « postérieur », « avant » et « arrière », « devant » et « derrière », « cranial » et « caudal », , « dessus » et « dessous », « supérieur(e) » et « inférieur(e) », « haut » et « bas », « latéral(e) » et « côté », « droit(e) » et « gauche », notamment, sont utilisés dans la suite en référence à cette convention. Ces termes correspondent aussi au vocabulaire utilisé par les personnes de métier dans le domaine de la chirurgie du rachis.

Le corps de la cale de stabilisation 1 comprend, d'un côté latéral du parallélépipède, plus particulièrement à droite sur les Figures 2 et 3, une échancrure ou encoche supérieure 15 et une échancrure ou encoche inférieure 16. Ces échancrures sont adaptées pour venir en appui contre deux vertèbres à stabiliser, et plus particulièrement sur l'apophyse de la vertèbre du dessus par l'encoche 15 et sur l'apophyse de la vertèbre du dessous par l'encoche 16, respectivement. Dit autrement, dans la position installée de l'implant pour assurer la stabilisation de deux vertèbres adjacentes, les apophyses épineuses de ces vertèbres se logent dans les échancrures 15 et 16 du corps de la cale 1.

L'évidement 12 est débouchant (ouvert) au moins du côté de la face supérieure la de la cale de stabilisation, et de préférence du côté de chacune des faces supérieure et inférieure de la cale.

Dans la zone d'entrée de l'évidement, les parois présentent un filetage intérieur (taraudage) 11.

La cale de stabilisation 1 comprend des passage 13, 14 et 17 traversant le corps 1 de part en part perpendiculairement à l'axe principal 10 du corps 1 de la cale. L'un au moins des passages 13 et 14, et de préférence les deux passages 13 et 14 débouchent dans l'évidement 12. Dans ce mode de réalisation représenté, les deux passages 13 et 14 passent par l'évidement 12 mais cela n'est pas obligatoire.

La cale de stabilisation est connue de l'état de la technique et décrite dans la demande de brevet FR1651203.

Selon la présente invention, quatre tailles de cale de stabilisation sont envisageables. Pour ces quatre tailles, la hauteur et la longueur de la cale seront invariables. Deux dimensions (A) et (B) telles que représentées sur la Figure 3, seront variables.

Typiquement, les dimensions seront les suivantes :

| | | |
|---|---|---|
| Taille 6 | A = 6mm | B = 16mm |
| Taille 8 | A = 8mm | B = 18mm |
| Taille 10 | A = 10mm | B = 20mm |
| Taille 12 | A = 12mm | B = 22mm |

Le matériau utilisé pour la cale est un polymère : le PolyEtherEtherKetone (PEEK).

Une fois la taille de la cale 1 choisie, le porte-implant 3 correspondant à la taille de cette cale sera utilisé et fixé par vissage à ladite cale de stabilisation, de manière à ce que le dispositif de renvoi 4 épouse la cale de stabilisation et que l'axe longitudinal 30 du porte-implant 3 coïncide avec l'axe longitudinal de l'évidement 12.

En effet, le porte-implant 3 de l'ensemble d'assistance présente à sa première extrémité 31 un filet adapté pour coopérer avec le taraudage de la zone d'entrée 11 de l'évidement 12 afin de permettre la fixation par vissage du porte-implant 3 à ladite cale de stabilisation de manière à ce que l'axe longitudinal 30 du porte-implant 3 coïncide avec l'axe longitudinal de l'évidement 12 et que la paroi de guidage 43 du dispositif de renvoi 4 comportant ladite ouverture 431 s'étende dans un plan moyen qui est sensiblement parallèle audit axe longitudinal 30 mais qui ne contient pas ledit axe longitudinal 30, et tel qu'illustré sur la Figure 1 ou 2.

La première paroi 41 du dispositif de renvoi 4, solidaire du porte-implant 3 et s'étendant perpendiculairement à l'axe longitudinal 30 du porte-implant est plaquée sur la face supérieure de la cale de stabilisation 1 de l'implant et la deuxième paroi 42, sensiblement perpendiculaire à la première paroi 41 est plaquée sur la face latérale 1b de la cale de stabilisation 1 de manière à bloquer en position et à mettre en porte-à-faux la paroi de guidage 43 par rapport audit implant.

Le deuxième bord 43d de la face arrière de la paroi de guidage 43 est plaqué contre le bord rostral de la cale de stabilisation de manière à ce que le renflement 432 du deuxième bord 43d de la face avant soit en saillie vers l'avant, tel que représenté à la Figure 5.

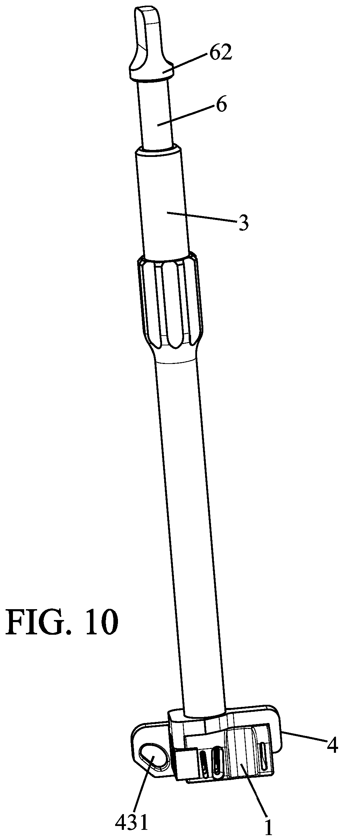

Le porte-implant s'utilise conjointement avec un verrouilleur d'implant (6). ayant un corps allongé s'étendant suivant un axe longitudinal entre des première et deuxième extrémités 61, 62, ledit verrouilleur étant adapté pour coulisser dans le canal interne 32 du corps tubulaire du porte implant 3 tel que représenté à la Figure 10.

La première extrémité 61 du verrouilleur d'implant présente à sa première extrémité un filet adapté pour coopérer avec le taraudage 11 de la zone d'entrée de l'évidemment afin que le verrouilleur d'implant 6 soit vissé jusqu'en butée dans la cale 1 afin de sécuriser l'assemblage du porte-implant 3 et de la cale de stabilisation 1 de manière à ce que l'axe longitudinal du porte implant coïncide avec l'axe longitudinal de l'évidemment et ce durant toute la durée de l'opération.

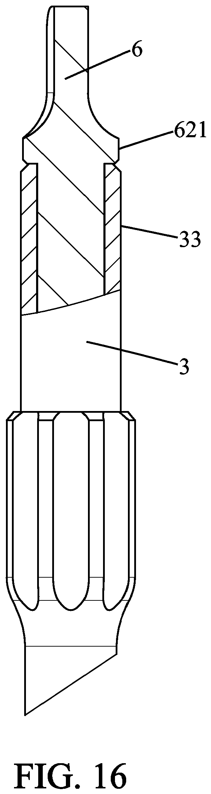

La seconde extrémité 62 du porte-implant 3 comporte un épaulement 621 tel que représenté à la Figure 16. L'épaulement 621 permet au verrouilleur d'implant 6 de prendre appui sur l'extrémité 33 du porte-implant afin de sécuriser l'assemblage du porte-implant 3 et de la cale de stabilisation 1 lorsque le verrouilleur d'implant 6 est vissé jusqu'en butée dans la cale 1.

Une fois le porte-implant 3 verrouillé en position sur la cale de stabilisation 1 par le verrouilleur d'implant 6, la tresse va pouvoir être insérée dans la cale de stabilisation 1. Le lien souple 2 peut être une tresse réalisée dans un matériau textile à usage médical (non résorbable), par exemple en Polyéthylène téréphtalate (PET) ou en Polyéthylène (PE). Ces matériaux peuvent être choisis en raison de leur biocompatibilité et de leur grande inertie chimique.

La longueur de la tresse est d'environ 700 millimètres, sa largeur d'environ 7 millimètre et son épaisseur d'environ 1.2 millimètres. Sa résistance à la traction est d'environ 170daN.

La première extrémité 21 du lien souple 2 est insérée manuellement par exemple d'abord au travers du passage 13, puis l'extrémité 21 est insérée au travers du passage 17.

Afin de passer la tresse du passage 13 au passage 17 à travers le ligament interépineux sus-jacent, un crochet peut être utilisé pour permettre le passage à travers le ligament interépineux.

Ainsi, l'extrémité 21 de la tresse est entrainée dans le sens des aiguilles d'une montre à travers le ligament interépineux, autour de l'apophyse épineuse et au plus près que possible de l'arête osseuse. Lorsque l'extrémité 21 de la tresse réapparait dans l'espace interépineux, elle peut être saisie, par exemple grâce à une pince à tresse.

Le crochet d'insertion est ensuite retiré et l'extrémité 21 de la tresse est tirée à travers le ligament pour être introduite dans le passage 17 de la cale de stabilisation 1.

La procédure est répétée comme précédemment décrit de manière à ce que la tresse passe autour de la deuxième épineuse tout en s'assurant qu'elle est positionné bien à plat contre l'épineuse sans torsion.

L'extrémité 21 de la tresse est ensuite introduite dans le troisième passage 14 de la cale.

Ainsi, la tresse 2 forme alors une boucle dans un plan perpendiculaire à l'axe principal 10 de la cale la, avec de préférence deux ganses 2a et 2b respectivement localisées de part et d'autre de la cale dans ledit plan. Ces ganses 2a et 2b de la tresse textile sont adaptées pour venir en engagement chacune avec l'une respective des apophyses épineuses des deux vertèbres à stabiliser.

L'extrémité 22 comporte un bourrelet formé par couture de l'extrémité 22 de manière ce que l'extrémité 22 de la tresse 2 soit fixée à la cale de stabilisation, une fois l'extrémité 21 engagée au travers des passages 13, 17 et 14. L'extrémité 21 est libre. Ainsi, l'extrémité 22 constituée d'un bourrelet cousu est destinée à venir se bloquer dans la cale après son introduction dans l'orifice 13 de ladite cale.

Après la mise en place de la tresse 2, celle-ci va être mise en tension afin de stabiliser le positionnement de l'implant entre les apophyses épineuses des vertèbres grâce à l'ensemble d'assistance au positionnement.

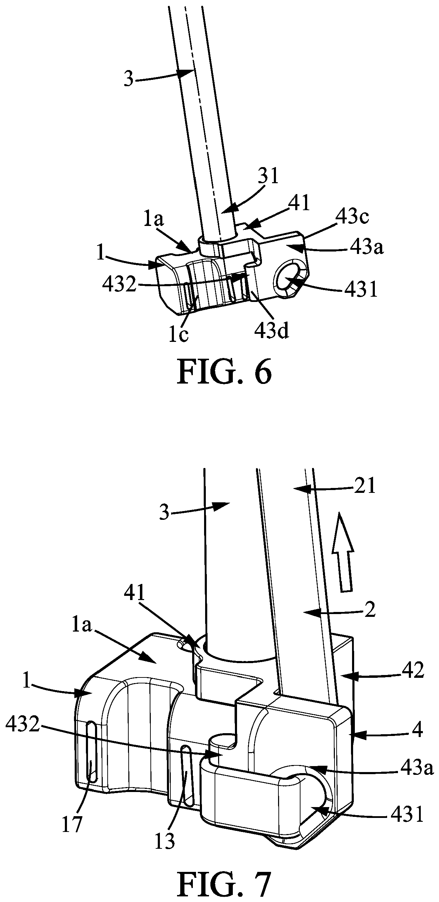

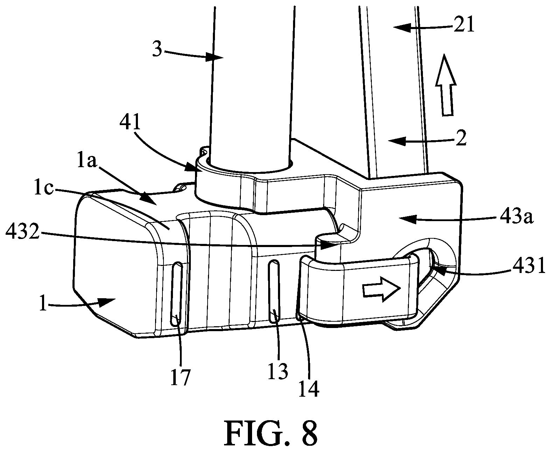

Comme illustré aux Figures 7 et 8, l'extrémité libre de la tresse 22 à sa sortie du passage 14 de la cale de stabilisation 1 va être renvoyée par le renflement 432 vers l'ouverture 431 de façon à ce que la tresse puisse présenter une première partie s'étendant sensiblement perpendiculairement à l'axe longitudinal 30 depuis ledit implant jusqu'à ladite ouverture, et une deuxième partie s'étendant depuis ladite ouverture jusqu'à ladite extrémité libre 21 dans un plan sensiblement parallèle à l'axe longitudinal 30 du porte-implant 3 pour permettre la mise en tension de ladite tresse 2.

L'axe principal longitudinal du porte implant 3 coïncide avec l'axe de la voie d'abord postérieure (par opposition, par exemple, à un accès latéral, ces termes « postérieure » et « latérale » étant ceux du vocabulaire utilisé dans ce domaine de la chirurgie du rachis). La tresse peut ainsi être avantageusement mise en tension selon la voie d'abord postérieure. La zone d'intervention et d'insertion peut donc être réduite au strict minimum. La mise en tension dans un plan sensiblement parallèle à l'axe longitudinal du porte implant permet donc d'éviter le délabrement musculaire, contrairement à une mise en tension latérale.

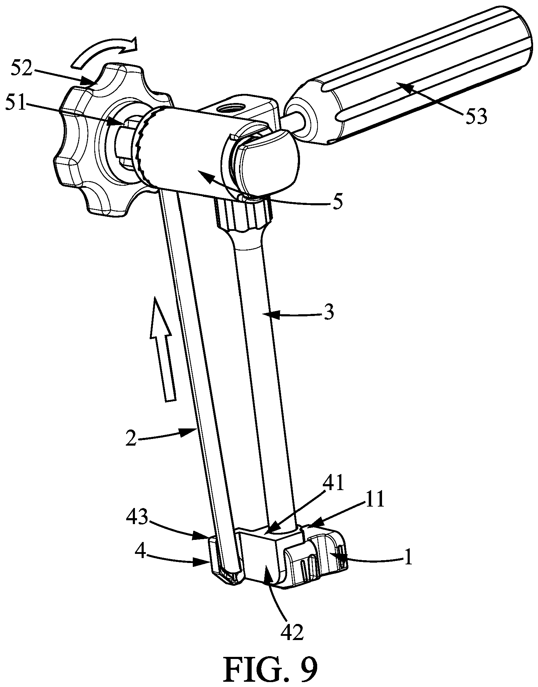

L'extrémité libre 22 va par la suite être reçue dans le tambour 51 du dispositif de mise en tension 5, tel que représenté à la Figure 9, et être mise en tension par enroulement de la tresse 2 sur le tambour 51 par actionnement de la poignée 52 permettant de faire tourner le tambour.

Avantageusement, la poignée 53 permet de maintenir en position l'ensemble d'assistance au positionnement de façon ergonomique.

La force induite par la traction lors de la mise en tension de la tresse 2 doit être estimée en utilisant un manche limiteur de couple (non représenté) qui vient se raccorder temporairement à la poignée 52 et coaxialement à son axe de rotation. Pour ce faire, un connecteur de limiteur de couple sert de liaison entre ledit manche limiteur de coupe et ladite poignée. L'opérateur maintient l'instrument à l'aide du manche 53 et actionne la poignée 52 en tournant le manche limiteur de couple dans le sens des aiguilles d'une montre pour ajuster la tension de la tresse autour des épineuses, jusqu'à atteindre la limite de couple, soit environ 6N.m. A ce stade, le manche limiteur de couple indique qu'une tension maximale de 300 Newtons est atteinte.

Une fois la tresse mise en tension, le verrouilleur d'implant peut être dévissé et retirée du canal interne 32 du porte-implant 3

Un pion de blocage ou verrou 7 est inséré dans l'évidement 12 de la cale de stabilisation 1 de manière à bloquer en mouvement par rapport à la cale de stabilisation par pincement ladite tresse entre le pion de blocage et les portions respectives de la paroi interne de l'évidement se faisant face l'une à l'autre. Ce blocage en mouvement est représenté aux Figures 13 à 15.

Avantageusement, le pincement de la tresse permet d'assurer son blocage une fois la tresse immobilisée en position avec la tension adéquate sans risque d'endommagement des fibres de la tresse.

La méthode de mise en place du pion de blocage 7 fait appel à une tige d'insertion du pion de blocage ayant une première extrémité et une seconde extrémité adaptée pour coulisser dans le canal interne 32 du corps tubulaire du porte-implant 3 pour l'insertion et le guidage du pion de blocage 7 à travers le canal interne 32 du porte-implant 3 jusque dans l'évidement 12 prévu dans le corps de la cale de stabilisation.

Une fois la tresse 2 bloquée en mouvement par le pion ou verrou, l'ensemble d'assistance au positionnement est désolidarisé de la cale 1 de l'implant qui est déjà ainsi en situation de stabilisation des vertèbres et la longueur de tresse 2 supplémentaire coupée.

Dans un mode de réalisation, le verrou ou pion de blocage 7 est d'un seul tenant, tel que représenté aux Figures 13 à 15 et est de forme sensiblement conique.

Le verrou est, par exemple, en alliage de titane (TA6V4 ISO 5832/3).

Une des extrémités du pion de blocage ou verrou 72 est de forme conique et permet, une fois le verrou vissé en butée, de pincer la tresse entre l'extrémité conique 72 du verrou 7 et les portions respectives de la paroi interne de l'évidement 12, se faisant face l'une à l'autre, de la cale de stabilisation.

Le pion de blocage 7 comprend, à l'opposé de son extrémité conique 72, une extrémité 71 présentant un filet adapté pour coopérer avec le taraudage 11 de la zone d'entrée de l'évidement 12 afin de visser ledit pion de blocage dans l'évidement de la cale de stabilisation jusqu'en butée.

Dans un autre mode de réalisation (non représenté), le verrou ou pion de blocage comprend deux parties. Une première partie correspondant au pion de blocage, de forme conique qui permet, une fois le verrou 1 vissé en butée, de pincer la tresse entre l'extrémité conique du verrou et les portions respectives de la paroi interne de l'évidement 12, se faisant face l'une à l'autre, de la cale de stabilisation.

Une deuxième partie correspondant à une vis de verrouillage qui peut être engagée dans la zone taraudée 11 de l'évidement 12 prévu dans la cale 1 afin de visser le pion de blocage.

Le pion de blocage et la vis de verrouillage ont également été décrit dans la demande de brevet FR1651203.

Avantageusement, l'ensemble d'assistance au positionnement de l'implant intervertébral permet une mise en place de la tresse ainsi que sa mise en tension selon une voie d'abord postérieure uniquement permet de réduire la taille de l'incision au strict minimum et ainsi de conserver l'intégrité des tissus organiques environnants, notamment les muscles du dos.

L'invention a été décrite et illustrée dans la présente description détaillée et dans les Figures, dans des formes de réalisation particulièrement avantageuses. Elle ne se limite pas, toutefois aux formes de réalisation présentées.

Dans les revendications, les termes « comprend » ou « comporte » n'excluent pas d'autres éléments ou d'autres étapes. Les différentes caractéristiques présentées et/ou revendiquées peuvent être avantageusement combinées. Leur présence dans la description ou dans des revendications dépendantes différentes, n'excluent pas cette possibilité. Les signes de référence ne sauraient être compris comme limitant la portée de l'invention.

## Revendications

1. Ensemble d'assistance au positionnement d'un implant intervertébral,
ledit implant comprenant une cale de stabilisation (1) adaptée pour stabiliser entre elles au moins deux vertèbres adjacentes par interposition entre des apophyses épineuses des vertèbres, et au moins une tresse (2) souple pour la fixation de la cale de stabilisation aux apophyses épineuses des vertèbres à stabiliser, ladite tresse (2) comprenant une extrémité (21) libre,
ledit ensemble comprenant un porte-implant (3) ayant un corps allongé s'étendant suivant un axe longitudinal (30) entre des première et deuxième extrémités (31, 33), la première extrémité étant adaptée pour être fixée à la cale de stabilisation (1),
**caractérisé en ce que** ladite première extrémité (31) dudit porte-implant comporte un dispositif de renvoi (4) comprenant une ouverture (431) ayant un axe central sensiblement perpendiculaire à l'axe longitudinal (30) du porte-implant (3) mais non-concourant avec ledit axe longitudinal (30), ladite ouverture (431) permettant ainsi le passage de ladite tresse (2) de façon que, en utilisation, ladite tresse (2) puisse présenter un première partie s'étendant sensiblement perpendiculairement à l'axe longitudinal (30) depuis ledit implant jusqu'à ladite ouverture, et une deuxième partie s'étendant depuis ladite ouverture jusqu'à ladite extrémité libre (21) dans un plan sensiblement parallèle à l'axe longitudinal (30) du porte-implant (3) pour permettre la mise en tension de ladite tresse (2).

2. Ensemble selon la revendication 1, dans lequel le dispositif de renvoi (4) comprend une paroi de guidage (43) qui comporte ladite ouverture (431), ladite paroi de guidage (43) s'étendant dans un plan moyen qui est sensiblement parallèle audit axe longitudinal (30) mais qui ne contient pas ledit axe longitudinal (30).

3. Ensemble selon la revendication 2, dans lequel ladite paroi de guidage comporte une face avant (43a) orientée généralement à l'opposé du porte-implant (3), ladite face avant s'étendant latéralement entre un premier bord relativement éloigné du porte-implant (3) et un deuxième bord plus proche du porte-implant (3), le deuxième bord de la face avant étant pourvu d'un renflement (432) en saillie vers l'avant, qui permet de renvoyer ladite première partie de la tresse vers l'ouverture (431).

4. Ensemble selon la revendication 3, dans lequel ledit système de renvoi (4) comprend en outre un support (41, 42) solidaire du porte-implant (3) et portant la paroi de guidage (43) en porte-à-faux par rapport audit porte-implant (3).

5. Ensemble selon la revendication 4, dans lequel ledit support comporte une première paroi (41) solidaire du porte-implant (3) et s'étendant sensiblement perpendiculairement audit axe longitudinal (30), et une deuxième paroi (42) reliant la première paroi (41) à la paroi de guidage (43), ladite deuxième paroi (42) étant sensiblement perpendiculaire à la première paroi (41) et à la paroi de guidage (43), ledit renflement s'étendant sensiblement à l'opposé de la deuxième paroi (42).

6. Ensemble selon l'une quelconque des revendications 1 à 5, dans lequel le corps allongé du porte-implant est de forme tubulaire avec un canal interne (32) s'étendant entre les première et deuxième extrémités (31), (32) suivant l'axe longitudinal (30) du corps allongé du porte-implant.

7. Ensemble selon l'une quelconque des revendications 1 à 6, dans lequel ladite deuxième extrémité (33) du porte-implant (3) comprend un dispositif de mise en tension (5) permettant de mettre en tension ladite tresse (2).

8. Ensemble selon la revendication 7, dans lequel ledit dispositif de mise en tension (5) comprend un tambour (51) configuré pour enrouler ladite tresse et une poignée (52) permettant de faire tourner ledit tambour.

9. Ensemble selon l'une quelconque des revendications 7 à 8, dans lequel ladite deuxième extrémité (33) du porte-implant (3) comporte en outre un manche (53).

10. Kit chirurgical comprenant un implant intervertébral et un ensemble d'assistance au positionnement selon l'une quelconque des revendications 1 à 9, ledit implant comprenant
- une cale de stabilisation (1) adaptée pour stabiliser entre elles au moins deux vertèbres adjacentes par interposition entre des apophyses épineuses des vertèbres, ayant un corps sensiblement parallélépipédique et comprenant une face supérieure (1a) et au moins une partie latérale (1b) présentant un axe principal déterminé (10), dans lequel est prévu un évidement (12) présentant un axe longitudinal déterminé parallèle à l'axe principal (10) du corps de la cale de stabilisation, une paroi interne s'étendant parallèlement à l'axe longitudinal de l'évidement, et une zone d'entrée taraudée (11);
- au moins une tresse (2) formant un lien souple pour la fixation de la cale de stabilisation aux apophyses épineuses des vertèbres à stabiliser, ladite au moins une tresse (2) présentant une première extrémité (22) fixée à ladite cale de stabilisation (1) et une seconde extrémité (21) libre.

11. Kit chirurgical selon la revendication 10, dans lequel le porte-implant (3) présente à sa première extrémité (31) un filet adapté pour coopérer avec le taraudage de la zone d'entrée (11) de l'évidement pour la fixation par vissage du porte-implant (3) à ladite cale de stabilisation (1) de manière à ce que l'axe longitudinal du porte-implant (3) coïncide avec l'axe longitudinal de l'évidement (12) et que la paroi de guidage (43) du dispositif de renvoi (4) comportant ladite ouverture (431) s'étende dans un plan moyen qui est sensiblement parallèle audit axe longitudinal (30) mais qui ne contient pas ledit axe longitudinal (30), de façon à ce que la tresse (2) lors de son passage dans l'ouverture (431) puisse présenter une première partie s'étendant sensiblement perpendiculairement à l'axe longitudinal (30) depuis ledit implant jusqu'à ladite ouverture (431), et une deuxième partie s'étendant depuis ladite ouverture jusqu'à ladite extrémité libre (21) dans un plan sensiblement parallèle à l'axe longitudinal (30) du porte-implant (3) pour permettre la mise en tension de ladite tresse (2).

12. Kit chirurgical selon les revendications 10 ou 11 combinée avec la revendication 3, dans lequel le deuxième bord de la face arrière de la paroi de guidage (43) est plaqué contre le bord rostral de la cale de stabilisation, de manière à ce que le renflement (432) du deuxième bord de la face avant de la paroi de guidage (43) soit en saillie vers l'avant, de façon à renvoyer ladite première partie de la tresse vers l'ouverture (431).

13. Kit chirurgical selon l'une quelconque des revendications 10 à 12 comprenant en outre une tige d'insertion d'un pion de blocage ayant une première extrémité et une seconde extrémité adaptée pour coulisser dans le canal interne (32) du corps tubulaire du porte-implant (3) pour l'insertion et le guidage du pion de blocage (7) à travers le canal interne (32) du porte-implant (3) jusque dans l'évidement (12) prévu dans le corps de la cale de stabilisation de manière à bloquer en mouvement par rapport à la cale de stabilisation par pincement ladite tresse entre le pion de blocage et les portions respectives de la paroi interne de l'évidement se faisant face l'une à l'autre, lorsque ladite tresse (2) a été mise en tension via le dispositif de mise en tension (5) de l'ensemble d'assistance au positionnement.

14. Kit chirurgical selon l'une quelconque des revendications 10 à 13 **caractérisé en ce qu'**il comprend un pion de blocage (7) d'un seul tenant ayant une forme conique ou sensiblement conique.

15. Kit chirurgical selon l'une quelconque des revendications 10 à 14 **caractérisé en ce qu'**il comprend une seconde cale de stabilisation, ladite ou un desdites tresses ayant une dimension longitudinale permettant son insertion dans les deux cales de stabilisation lorsque chacune de ces cales est placée entre deux apophyses.

## Patentansprüche

1. Anordnung zur Unterstützung bei der Positionierung eines Zwischenwirbel-Implantats, wobei das Implantat einen Stabilisierungskeil (1) umfasst, der dazu angepasst ist, mindestens zwei benachbarte Wirbel durch Zwischenschaltung zwischen Dornfortsätzen der Wirbel zwischen einander zu stabilisieren, und mindestens ein flexibles Geflecht (2) zur Fixierung des Stabilisierungskeils an den Dornfortsätzen der zu stabilisierenden Wirbel, wobei das Geflecht (2) ein freies Ende (21) umfasst,
wobei die Anordnung einen Implantat-Halter (3) mit einem länglichen Körper umfasst, der sich entlang einer Längsachse (30) zwischen ersten und zweiten Enden (31, 33) erstreckt, wobei das erste Ende dazu angepasst ist, an dem Stabilisierungskeil (1) befestigt zu werden,
**dadurch gekennzeichnet, dass** das erste Ende (31) des Implantat-Halters eine Rückführvorrichtung (4) umfasst, die eine Öffnung (431) umfasst, mit einer Mittelachse, die im Wesentlichen senkrecht zur Längsachse (30) des Implantat-Halters (3) verläuft, jedoch nicht mit der Längsachse (30) zusammentrifft, wobei die Öffnung (431) so den Durchgang des Geflechts (2) ermöglicht, so dass im Einsatz das Geflecht (2) einen ersten Teil aufweisen kann, der sich im Wesentlichen senkrecht zur Längsachse (30) von dem Implantat bis zu der Öffnung erstreckt, und einen zweiten Teil aufweisen kann, der sich von der Öffnung zu dem freien Ende (21) in einer Ebene erstreckt, die im Wesentlichen parallel zur Längsachse (30) des Implantat-Halters (3) verläuft, um das Spannen des Geflechts (2) zu ermöglichen.

2. Anordnung nach Anspruch 1, wobei die Rückführvorrichtung (4) eine Führungswand (43) umfasst, welche die Öffnung (431) umfasst, wobei sich die Führungswand (43) in einer mittleren Ebene erstreckt, die im Wesentlichen parallel zur Längsachse (30) verläuft, die Längsachse (30) jedoch nicht enthält.

3. Anordnung nach Anspruch 2, wobei die Führungswand eine Vorderseite (43a) umfasst, die im Allgemeinen gegenüber dem Implantat-Halter (3) ausgerichtet ist, wobei sich die Vorderseite seitlich zwischen einer ersten Kante, die relativ weit vom Implantat-Halter (3) entfernt ist, und einer zweiten Kante näher am Implantat-Halter (3) erstreckt, wobei die zweite Kante der Vorderseite mit einer nach vorne ragenden Ausbuchtung (432) versehen ist, die es ermöglicht, den ersten Teil des Geflechts in Richtung der Öffnung (431) zurückzubringen.

4. Anordnung nach Anspruch 3, wobei das Rückführsystem (4) ferner einen Träger (41, 42) umfasst, der mit dem Implantat-Halter (3) integriert ist und die Führungswand (43) auskragend relativ zu dem Implantat-Halter (3) trägt.

5. Anordnung nach Anspruch 4, wobei der Träger eine erste Wand (41) umfasst, die mit dem Implantat-Halter (3) integriert ist und sich im Wesentlichen senkrecht zu der Längsachse (30) erstreckt, und eine zweite Wand (42) umfasst, welche die erste Wand (41) mit der Führungswand (43) verbindet, wobei die zweite Wand (42) im Wesentlichen senkrecht zur ersten Wand (41) und zur Führungswand (43) verläuft, wobei sich die Ausbuchtung im Wesentlichen gegenüber der zweiten Wand (42) erstreckt.

6. Anordnung nach einem der Ansprüche 1 bis 5, wobei der längliche Körper des Implantat-Halters eine röhrenförmige Form mit einem inneren Kanal (32) aufweist, der sich zwischen dem ersten (31) und dem zweiten Ende (32) erstreckt, entlang der Längsachse (30) des länglichen Körpers des Implantat-Halters.

7. Anordnung nach einem der Ansprüche 1 bis 6, wobei das zweite Ende (33) des Implantat-Halters (3) eine Spannvorrichtung (5) zum Spannen des Geflechts (2) umfasst.

8. Anordnung nach Anspruch 7, wobei die Spannvorrichtung (5) eine Trommel (51), die zum Aufwickeln des Geflechts konfiguriert ist, und einen Griff (52) umfasst, der es ermöglicht, die Trommel zu drehen.

9. Anordnung nach einem der Ansprüche 7 bis 8, wobei das zweite Ende (33) des Implantat-Halters (3) ferner einen Stiel (53) umfasst.

10. Chirurgisches Kit, umfassend ein Zwischenwirbel-Implantat und eine Anordnung zur Unterstützung bei der Positionierung gemäß einem der Ansprüche 1 bis 9, wobei das Implantat umfasst:
- einen Stabilisierungskeil (1), der dazu angepasst ist, mindestens zwei benachbarte Wirbel durch Zwischenschaltung zwischen Dornfortsätzen der Wirbel zwischen einander zu stabilisieren, der einen im Wesentlichen parallelepipedförmigen Körper aufweist und eine Oberseite (1a) und mindestens einen Seitenteil (1b) umfasst, mit einer bestimmten Hauptachse (10), in dem eine Aussparung (12) vorgesehen ist, die eine bestimmte Längsachse parallel zur Hauptachse (10) des Körpers des Stabilisierungskeils aufweist, eine Innenwand, die sich parallel zur Längsachse der Aussparung erstreckt; und einen Gewindeeingangsbereich (11);
- mindestens ein Geflecht (2), das eine flexible Verbindung zur Fixierung des Stabilisierungskeils an den Dornfortsätzen der zu stabilisierenden Wirbel bildet, wobei das mindestens eine Geflecht (2) ein erstes Ende (22), das an dem Stabilisierungskeil (1) befestigt ist, und ein zweites, freies Ende (21) aufweist.

11. Chirurgisches Kit nach Anspruch 10, wobei der Implantat-Halter (3) an seinem ersten Ende (31) ein Gewinde aufweist, das dazu angepasst ist, mit dem Gewindeeingangsbereich (11) der Aussparung zur Fixierung des Implantat-Halters (3) an dem Stabilisierungskeil (1) durch Verschrauben zusammenzuwirken, so dass die Längsachse des Implantat-Halters (3) mit der Längsachse der Aussparung (12) zusammenfällt, und die Führungswand (43) der Rückführvorrichtung (4), welche die Öffnung (431) aufweist, sich in einer mittleren Ebene erstreckt, die im Wesentlichen parallel zur Längsachse (30) ist, aber die Längsachse (30) nicht enthält, so dass das Geflecht (2) bei seinem Durchgang durch die Öffnung (431) einen ersten Teil aufweisen kann, der sich im Wesentlichen senkrecht zur Längsachse (30) von dem Implantat zu der Öffnung (431) erstreckt, und einen zweiten Teil, der sich von der Öffnung zu dem freien Ende (21) in einer zur Längsachse (30) des Implantat-Halters (3) im Wesentlichen parallelen Ebene erstreckt, um das Spannen des Geflechts (2) zu ermöglichen.

12. Chirurgisches Kit nach Anspruch 10 oder 11 in Kombination mit Anspruch 3, wobei die zweite Kante der Rückseite der Führungswand (43) gegen die rostrale Kante des Stabilisierungskeils gedrückt wird, so dass die Ausbuchtung (432) der zweiten Kante der Vorderseite der Führungswand (43) nach vorne vorsteht, um den ersten Teil des Geflechts zur Öffnung (431) zurückzubringen.

13. Chirurgisches Kit nach einem der Ansprüche 10 bis 12, ferner umfassend einen Stab zum Einsetzen eines Verriegelungsstifts mit einem ersten Ende und einem zweiten Ende, angepasst zum Gleiten in dem inneren Kanal (32) des röhrenförmigen Körpers des Implantat-Halters (3) zum Einsetzen und Führen des Verriegelungsstifts (7) durch den inneren Kanal (32) des Implantat-Halters (3) bis in die Aussparung (12), die in dem Körper des Stabilisierungskeils vorgesehen ist, um die Bewegung relativ zum Stabilisierungskeil durch Einklemmen des Geflechts zwischen dem Verriegelungsstift und den jeweiligen Teilen der Innenwand der Aussparung, die einander zugewandt sind, zu blockieren, wenn das Geflecht (2) durch die Spannvorrichtung (5) der Anordnung zur Unterstützung bei der Positionierung gespannt wurde.

14. Chirurgisches Kit nach einem der Ansprüche 10 bis 13, **dadurch gekennzeichnet, dass** es einen einstückigen Verriegelungsstift (7) mit einer konischen oder im Wesentlichen konischen Form umfasst.

15. Chirurgisches Kit nach einem der Ansprüche 10 bis 14, **dadurch gekennzeichnet, dass** es einen zweiten Stabilisierungskeil umfasst, wobei das eine Geflecht oder eines der Geflechte eine Längsabmessung aufweist, die sein Einsetzen in die beiden Stabilisierungskeile ermöglicht, wenn jeder dieser Keile zwischen zwei Fortsätzen angeordnet ist.

## Claims

1. Assembly for assisting with the positioning of an intervertebral implant,
said implant comprising a stabilization wedge (1) suitable for stabilizing at least two adjacent vertebrae relative to each other by interposition between spinous processes of the vertebrae, and at least one flexible braid (2) for fixing the stabilization wedge to the spinous processes of the vertebrae that are to be stabilized, said braid (2) comprising a free end (21),
said assembly comprising an implant holder (3) having an elongate body extending along a longitudinal axis (30) between first and second ends (31, 33), the first end being suitable for being fixed to the stabilization wedge (1) ,
**characterized in that** said first end (31) of said implant holder has a deflection device (4) comprising an opening (431) having a central axis substantially perpendicular to the longitudinal axis (30) of the implant holder (3) but not intersecting said longitudinal axis (30), said opening (431) thus allowing said braid (2) to pass through it in such a way that, when in use, said braid (2) can have a first part extending substantially perpendicularly with respect to the longitudinal axis (30) from said implant to said opening, and a second part extending from said opening to said free end (21) in a plane substantially parallel to the longitudinal axis (30) of the implant holder (3) in order to permit the tensioning of said braid (2).

2. Assembly according to Claim 1, in which the deflection device (4) comprises a guide wall (43) having said opening (431), said guide wall (43) extending in a mean plane which is substantially parallel to said longitudinal axis (30) but which does not contain said longitudinal axis (30).

3. Assembly according to Claim 2, in which said guide wall has a front face (43a) oriented generally away from the implant holder (3), said front face extending laterally between a first edge relatively distant from the implant holder (3) and a second edge closer to the implant holder (3), the second edge of the front face being provided with a bulge (432) protruding toward the front, which makes it possible to deflect said first part of the braid toward the opening (431).

4. Assembly according to Claim 3, in which said deflection system (4) additionally comprises a support (41, 42) rigidly connected to the implant holder (3) and carrying the guide wall (43) jutting out from said implant holder (3).

5. Assembly according to Claim 4, in which said support has a first wall (41) connected to the implant holder (3) and extending substantially perpendicularly with respect to said longitudinal axis (30), and a second wall (42) joining the first wall (41) to the guide wall (43), said second wall (42) being substantially perpendicular to the first wall (41) and to the guide wall (43), said bulge extending substantially away from the second wall (42).

6. Assembly according to any one of Claims 1 to 5, in which the elongate body of the implant holder is of a tubular shape with an internal channel (32) extending between the first and second ends (31), (32) along the longitudinal axis (30) of the elongate body of the implant holder.

7. Assembly according to any one of Claims 1 to 6, in which said second end (33) of the implant holder (3) comprises a tensioning device (5) making it possible to tension said braid (2).

8. Assembly according to Claim 7, in which said tensioning device (5) comprises a drum (51) configured to wind said braid, and a handle (52) allowing said drum to be turned.

9. Assembly according to either of Claims 7 and 8, in which said second end (33) of the implant holder (3) additionally has a sleeve (53).

10. Surgical kit comprising an intervertebral implant and a positioning assistance assembly according to any one of Claims 1 to 9, said implant comprising:
- a stabilization wedge (1) suitable for stabilizing at least two adjacent vertebrae relative to each other by interposition between spinous processes of the vertebrae, having a substantially parallelepipedal body and comprising an upper face (1a) and at least one lateral part (1b) with a defined main axis (10), in which there is provided a recess (12) with a defined longitudinal axis parallel to the main axis (10) of the body of the stabilization wedge, an inner wall extending parallel to the longitudinal axis of the recess, and an internally threaded inlet zone (11);
- at least one braid (2) forming a flexible link for fixing the stabilization wedge to the spinous processes of the vertebrae that are to be stabilized, said at least one braid (2) having a first end (22), fixed to said stabilization wedge (1), and a free second end (21).

11. Surgical kit according to Claim 10, in which the implant holder (3) has, at its first end (31), a thread suitable for cooperating with the internal thread of the inlet zone (11) of the recess in order to fix the implant holder (3) to said stabilization wedge (1) by screwing, in such a way that the longitudinal axis of the implant holder (3) coincides with the longitudinal axis of the recess (12) and that the guide wall (43) of the deflection device (4) having said opening (431) extends in a mean plane which is substantially parallel to said longitudinal axis (30) but which does not contain said longitudinal axis (30), in such a way that the braid (2), during its passage through the opening (431), can have a first part extending substantially perpendicularly with respect to the longitudinal axis (30) from said implant to said opening (431), and a second part extending from said opening to said free end (21) in a plane substantially parallel to the longitudinal axis (30) of the implant holder (3) in order to permit the tensioning of said braid (2).

12. Surgical kit according to Claim 10 or 11 in combination with Claim 3, in which the second edge of the rear face of the guide wall (43) is placed flat against the rostral edge of the stabilization wedge, in such a way that the bulge (432) of the second edge of the front face of the guide wall (43) protrudes forward, in such a way as to deflect said first part of the braid toward the opening (431).

13. Surgical kit according to any one of Claims 10 to 12, additionally comprising an insertion rod for a blocking pin having a first end and a second end suitable for sliding in the internal channel (32) of the tubular body of the implant holder (3) for the insertion and guiding of the blocking pin (7) through the internal channel (32) of the implant holder (3) as far as the recess (12) provided in the body of the stabilization wedge, in such a way as to block movement with respect to the stabilization wedge by clamping said braid between the blocking pin and the respective mutually facing portions of the inner wall of the recess, when said braid (2) has been tensioned via the tensioning device (5) of the positioning assistance assembly.

14. Surgical kit according to any one of Claims 10 to 13, **characterized in that** it comprises a one-piece blocking pin (7) having a conical or substantially conical shape.

15. Surgical kit according to any one of Claims 10 to 14, **characterized in that** it comprises a second stabilization wedge, said braid or one of said braids having a longitudinal dimension permitting its insertion in the two stabilization wedges when each of these wedges is placed between two spinous processes.
